# EUROPÄISCHE PATENTSCHRIFT

(11) **EP 1 883 699 B1**
(45) Veröffentlichungstag und Bekanntmachung des Hinweises auf die Patenterteilung: **04.07.2012**
(21) Anmeldenummer: 06753191.3
(22) Anmeldetag: 20.05.2006
(51) Int. Cl.: A61K 48/00, A61K 47/48, C12N 15/63, C08G 81/00, A61K 38/00

(54) **NICHT VIRALES VEKTORSYSTEM ZUM TRANSPORT VON NUKLEINSÄURE IN DIE LUNGE**
NON-VIRAL VECTOR SYSTEM FOR TRANSPORTING NUCLEIC ACID INTO THE LUNGS
SYSTEME DE VECTEURS NON VIRAUX POUR LE TRANSPORT D'ACIDE NUCLEIQUE DANS LES POUMONS

(30) Priorität: 20.05.2005 DE 102005023993
(43) Veröffentlichungstag der Anmeldung: 06.02.2008
(73) Patentinhaber: Transmit Gesellschaft für Technologietransfer mbH, 35394 Giessen (DE); Justus-Liebig-Universität Gießen, 35390 Giessen (DE); PHILIPPS-UNIVERSITÄT MARBURG, 35037 Marburg (DE)
(72) Erfinder: KISSEL, Thomas, 79219 Staufen (DE); KLEEMANN, Elke, 55124 Mainz-Gonsenheim (DE); NEU, Michael, 68535 Edingen-Neckarhausen (DE); SCHMEHL, Thomas, 35390 Giessen (DE)
(74) Vertreter: Buchhold, Jürgen
(86) Internationale Anmeldenummer: PCT/DE2006/000875
(87) Internationale Veröffentlichungsnummer: WO 2006/122542

(56) Entgegenhaltungen:
- WO-A-98/59064
- WO-A2-02/060412
- DE-A1- 10 015 906
- US-A1- 2004 248 842
- RUDOLPH CARSTEN ET AL: "Oligomers of the arginine-rich motif of the HIV-1 TAT protein are capable of transferring plasmid DNA into cells." JOURNAL OF BIOLOGICAL CHEMISTRY, Bd. 278, Nr. 13, 28. März 2003 (2003-03-28), Seiten 11411-11418, XP002403121 ISSN: 0021-9258
- HO A ET AL: "Synthetic protein transduction domains: enhanced transduction potential in vitro and in vivo" CANCER RESEARCH, AMERICAN ASSOCIATION FOR CANCER RESEARCH, BALTIMORE, MD, US, Bd. 61, 15. Januar 2001 (2001-01-15), Seiten 474-477, XP002179150 ISSN: 0008-5472
- JARVER PETER ET AL: "The use of cell-penetrating peptides as a tool for gene regulation" DRUG DISCOVERY TODAY, Bd. 9, Nr. 9, 1. Mai 2004 (2004-05-01), Seiten 395-402, XP002403122 ISSN: 1359-6446
- KLEEMANN ET AL: "Nano-carriers for DNA delivery to the lung based upon a TAT-derived peptide covalently coupled to PEG-PEI" JOURNAL OF CONTROLLED RELEASE, ELSEVIER, AMSTERDAM, NL, Bd. 109, Nr. 1-3, 5. Dezember 2005 (2005-12-05), Seiten 299-316, XP005204221 ISSN: 0168-3659
- NEU MICHAEL ET AL: "Recent advances in rational gene transfer vector design based on poly(ethylene imine) and its derivatives" JOURNAL OF GENE MEDICINE, Bd. 7, Nr. 8, August 2005 (2005-08), Seiten 992-1009, XP009073801 ISSN: 1099-498X

## Beschreibung

Die vorliegend Erfindung betrifft ein nicht-virales Vektorsystem für den Transport von Nukleinsäuren, das auf der Basis von Polyethylenimin (PEI) mit Polyethylenglycol (PEG) modifiziert ist und eine Peptidsequenz mit PTD/CPP- Funktionalität aufweist. Es ist aufgrund geringer Toxizität und guter Stabilität für den Transport von Nukleinsäuren in die Lunge und daher für die Behandlung von Lungenerkrankungen gut geeignet.

### Hintergrund

Eines der Schlüsselprobleme in der Gentherapie und der damit verbundenen Behandlung von Krankheiten mit genetischem Material ist das Auffinden eines geeigneten Transportmittels, um das genetische Material zu den entsprechenden Zielzellen zu bringen, damit es dort zur Expression kommt.

Die Lunge stellt ein wichtiges Organ an der Grenzfläche zwischen Umgebung und Körperinnerem dar. Gasförmige oder partikuläre Substanzen können über den leitend mit der Umgebung verbundenen Respirationstrakt in die verschiedenen Verzweigungen der Lunge gelangen und dort mit den begrenzenden Innenflächen des Atemtrakts interagieren. Insbesondere bei der Behandlung von Lungenerkrankungen wie pulmonaler Hypertonie, Mukoviszidose und Lungentumoren besteht großer Bedarf an einem Transportmittel für DNA.

Um in vivo einsetzbar zu sein, werden an ein geeignetes Vektorsystem generell besondere Anforderungen gestellt
- Gewebespezifität:
   das Vektorsystem muss Zielzell-spezifisch sein, besonders, wenn das Vektorsystem die zu exprimierenden Gene in nur einen bestimmten Zelltyp schleusen soll
- Größe der eingefügten DNA
   das Vektorsystem sollte eine unlimitierte Größe an einzusetzender DNA tolerieren und auch große Gene transportieren können
- Immunogenität
   das Vektorsystems sollte selbst nicht immunogen sein, keine Immunantwort in der transfizierten Zelle oder im Patienten auslösen
- Toxizität
   das Vektorsystem sollte selbst keine zytotoxischen Effekte auf die Zellen haben
- Stabilität
   das Vektorsystem muss stabil gegenüber Degradation durch Endo- und Exonukleasen sein, je nach Anwendungsgebiet auch stabil gegenüber der Anwendungsform, z.B. der Vemeblung
- Größe
   das Vektorsystem sollte möglichst klein sein, um in die Zelle und in oder an den Zellkern eingeschleust werden zu können
- Zellpenetration und Kemtransport
   das Vektorsystem sollte mit großer Transfektionseffizienz zur Zelle, in oder an den Zellkern gelangen
- Herstellbarkeit
   das Vektorsystem sollte für die kommerzielle Anwendung leicht und preiswert herzustellen, lagerbar und transportfähig sein.

Der Stand der Technik kennt Vektorsysteme in Form von viralen Vektoren, z.B. Retrovirale, Adenovirale Vektoren oder Adeno-assoziierte Vektoren, sowie nicht-virale Vektoren z.B. Polyethylenimine oder Dendrimere und die Applikation von nackter DNA mittels physikalischer Methoden wie z.B. Elektroporation oder "Gene gun".

Prinzipiell sind virale Vektoren als Transfektions- und Expressionsvektoren zum Transfer von Genen in Säugetierzellen sehr effizient, jedoch ist Ihre Aufnahmekapazität für therapeutische Fremd-DNA sehr begrenzt und sie weisen in unterschiedlichem Ausmaß stets immonogene Eigenschaften auf. Dies bedeutet, dass sie zwar Gene recht zielsicher übertragen, dabei aber die Zelle schädigen und starke Reaktionen des Immunsystems auslösen können.

Um dem Problem der Nebenwirkungen der Viren aus dem Weg zu gehen, haben Forscher nicht-virale Transportsysteme entwickelt. Nicht-virale Vektoren verwenden pure DNA, die für ein bestimmtes Protein kodiert und mit den nötigen Regulationselementen zur Expression dieser Proteinsequenz versehen ist. Diese pure DNA wird auf verschiedene Weise in die Zelle gebracht, nicht stabil ins Genom integriert, sondern verbleibt dort extrachromosomal und führt zu einer endogenen Synthese des Proteins. Alternativ werden die zu übertragenden Gene auch in Liposomen oder Polymere eingehüllt und können mittels Membranfusion bzw. Endozytose sicher in die Zellen transportiert werden. So genannte Lipoplexe werden heute bereits in der Therapie am Menschen eingesetzt z.B. bei der Behandlung von Mukoviszidose und dem schwarzen Hautkrebs (malignes Melanom). Die Verwendung von nackter DNA als Vektor hat den Vorteil, dass sie leicht genetisch manipulierbar ist, so dass sie in der Gentherapie eingesetzt werden kann.

Plasmide sind kleine extrachromosomale DNA-Stücke, die als Vektoren besonders gut geeignet sind, große Gene in eine Vielzahl von Zellen zu transportieren. Sie werden routinemäßig zum Gentransfer von einem zum anderen Organismus eingesetzt. Im Gegensatz zu viralen Vektoren können Plasmide in großen Mengen, guter Qualität und billig hergestellt werden.

Carsten et al. (Journal of Biological Chemistry, Bd. 278, Nr. 13, 28.03.2003, S. 11411-11418) offenbart Komplexe aus PEI, speziellen TAT-Peptiden und DNA. Bei diesen TAT-Peptiden handelt es sich um Variationen der Sequenz C(YGRKKRRQRRG)ₙ. Die dabei gebildeten Komplexe zeichnen sich durch einen relativ großen Partikeldurchmesser aus.

US 2003/238832 A1 offenbart Komplexe aus Polyethylenimin (PEI), die mit einem hydrophilen Polymer als bifunktionalem Linker modifiziert sind. Das Polyethylenimin kann dabei an einen zellulären Liganden, nämlich Transferrin gekoppelt sein.

WO 02/060412 A3 offenbart die gemeinsame Verwendung von Polyethylenglykol (PEG) und Polyethylenimin (PEI), nicht jedoch ein Peptid, welches eine Proteintransduktionsdomäne oder eine Zellpenetrationsdomäne aufweist.

Ho et al. (CancerResearch, American Association for cancer Research, Baltimore, MD, US, Bd. 61, 15.01.2011, Seiten 474-477) beschreiben synthetische Proteintransduktionsdomänen (PTD). Sie sieht jedoch nicht vor, dass diese Proteintransduktionsdomänen in Kombination mit Polyethylenglykol (PEG) und Polyethylenimin (PEI) als nicht-virales Vektorssystem dienen.

Aufgabe der Erfindung ist es, ein nicht-virales Vektorsystem für den Transport von Nukleinsäure bereitzustellen, das eine große Transfektionseffizienz und geringe Toxizität aufweist und für den Einsatz in der Lunge geeignet ist, sowie ein leicht durchführbares und kostengünstiges Herstellungsverfahren.

Diese Aufgabe wird gemäß Anspruch 1 gelöst, indem ein Vektorsystem für den Transport von Nukleinsäure mit Polyplexen aus Polyethylenimin (PEI), Polyethylenglycol (PEG) und einem Peptid mit PTD/CPP-Funktionalität bereitgestellt wird sowie ein Verfahren gemäß Anspruch 18 zu dessen Herstellung.

Besonders vorteilhafte Eigenschaften des erfindungsgemäßen Vektorsystems aus einem Peptid mit PTD/CPP-Funktionalität, PEG und PEI sind:
- verstärkte Stabilität und Schutz der eingeschlossenen Nukleinsäure vor allem in der pulmonalen Umgebung
- das Zeta-Potential und somit die Oberflächenladung der Polyplexe ist reduziert und führt zu einer geringen Zytotoxizität in-vitro und in-vivo beispielsweise an Lungenepithelzellen
- Polyplexe weisen eine verminderte Aggregationstendenz in Medium mit großer Ionenstärke auf
- 600% Anstieg der Transfektionseffizienz in vivo verglichen mit Polyethylenimin 25kDa (PEI)
- Transport von Nukleinsäure z.B. DNA, RNA und/oder Plasmid-DNA direkt in die epithelialen Zellen der Bronchien und Alveolen
- Anwendung in der lokalen Lungentherapie

Überraschenderweise wurde gefunden, dass Vektorsysteme auf der Basis von PEI unter in vivo-Bedingungen besonders gute Stabilität und hohe Transfektionseffizienz aufweisen, wenn sie über einen Linker Peptidsequenzen mit PTD/CPP-Funktionalität tragen.

Erfindungsgemäß wird hierbei unter einem Peptid mit PTD/CPP-Funktionalität ein Peptid mit einer Proteintransduktionsdomäne (PTD) oder einer Zellpenetrationsdomäne (CPP) verstanden. Vorzugsweise ist dies das TAT-Peptid oder eine dem TAT-Peptid verwandte Peptidsequenz. Das TAT-Peptid ist ein aus 86 Aminosäuren aufgebautes Membranpeptid aus dem HIV-Virus (human immune deficiency virus type 1).

Weitere Peptide mit einer Proteintransduktionsdomäne oder einer Zellpenetrationsdomäne sind bekannt, darunter MAP, Antp und MAT, sowie deren Derivate. Als Linker wird vorzugsweise Polyethylenglycol (PEG) eingesetzt, es ist aber auch möglich andere geeignete Linker einzusetzen.

Transfektion bedeutet allgemein das Einbringen von Nukleinsäure, insbesondere Fremd-DNA in Zellen, wobei das Einbringen von Nukleinsäure transient oder stabil sein kann.

Die Nukleinsäure kodiert bevorzugt für Substanzen, denen alleine oder in Kombination ein positiver Effekt bei der Behandlung von Lungenerkrankungen zugesprochen wird. Die Nukleinsäure kann dabei DNA oder RNA sein.

Die Synthese des Polymerkonjugates erfolgt, indem ein Oligopeptid mit PTD/CPP-Funktionalität am C-Terminus beispielsweise über einen Cysteinrest modifiziert und über den hetero-bifunktionalen Linker, beispielsweise PEG, kovalent an PEI gekoppelt wird.

Das erfindungsgemäße nicht-virale Vektorsystem zum Transport von Nukleinsäuren zeigt herausragende Eigenschaften zur Verwendung an der Lunge zur Behandlung von Patienten, die an Lungenerkrankungen leiden.

Der Begriff Patient bezieht sich dabei gleichermaßen auf Menschen und Wirbeltiere. Damit kann das erfindungsgemäße nicht-virale Vektorsystem zum Transport von Nukleinsäuren in der Human- und Veterinärmedizin verwendet werden. Vorzugsweise wird es zur Herstellung eines Mittels zur Behandlung von Lungenerkrankungen, wie pulmonaler Hypertonie, Mukoviszidose und Lungentumoren eingesetzt.

Das nicht-virale Vektorsystem der vorliegenden Erfindung wird den Patienten, als Teil einer pharmazeutisch akzeptablen Komposition entweder inhalativ, oral, rektal, parenteral intravenös, intramuskulär oder subkutan, intracisternal, intravaginal, intraperitoneal, intravasculär, lokal (Puder, Salbe oder Tropfen), über intratracheale Intubation, intratracheale Instillation oder in Sprayform verabreicht.

Pharmazeutisch akzeptable Kompositionen können die Modifikationen als Salze, Ester, Amide und "Prodrugs" beinhalten, sofern sie nach zuverlässiger medizinischer Beurteilung keine übermäßige Toxizität, Irritationen oder allergische Reaktionen am Patienten auslösen.

Der Terminus "Prodrug" bezieht sich auf Verbindungen, die zur Verbesserung der Aufnahme transformiert werden, wie beispielsweise durch Hydrolyse im Blut.

Dosierungsformen für die örtliche Administration dieser Erfindung schließen Salben, Puder, Sprays oder Inhalationsmittel ein. Die aktive Komponente wird unter sterilen Bedingungen, mit einem physiologisch akzeptablen Trägerstoff und möglichen Preservativen, Puffern oder Treibmitteln, je nach Bedarf, vermischt.

### Ausführungsbeispiel:

Das nicht-virale Vektorsystem zum Transport von Nukleinsäuren ist ein Polymerkonjugat aus PEG, PEI und Peptidsequenzen mit PTD/CPP-Funktionalität. Beispielhaft für ein Protein mit PTD/CPP-Funktionalität wird das TAT-Peptid oder eine dem TAT-Peptid verwandte Peptidsequenz verwendet.

Beispielhaft für eine TAT-Peptid verwandte Peptidsequenz ist die Decapeptidsequenz GRKKKRRQRC. Weitere verwandte TAT-Peptidsequenzen sind bekannt und können alternativ eingesetzt werden. Diese werden auftragsmäßig synthetisiert beispielsweise von der Firma Bachem.

### Weitere Materialien sind:

Plasmid pGL3, das eine Luziferase codierende Region aufweist und unter der Kontrolle des Promotor des Cytomegalovirus (CMV) steht, ist bei Promega GmbH zu kaufen und wird nach dem dort angegebenen Standardprotokoll in E. coli vermehrt, isoliert und gereinigt.

Hering Testes-DNA ist kommerziell erhältlich z.B. bei der Firma Sigma.

Plasmid peGFP-N1, das die green fluorescence coding region unter der Kontrolle des Promotor des Cytomegalovirus (CMV-N1) trägt, ist bei ClonTech (1290 Terra Bella Avenue Mountain View, CA 94043 USA) zu kaufen und wird nach dem dort angegebenen Standardprotokoll in E. coli vermehrt, isoliert und gereinigt. Das natürliche Surfactant Alveofact® ist kommerziell z.B. bei Boehringer-Ingelheim erhältlich. Bronchial alveolar lavage fluid (BALF) wird nach Standardprotokoll aus C57BL/6 Mäusen via intra-tracheal Instillation hergestellt. Zur Entfernung störender Zellen wird BALF zentrifugiert z.B. bei 300 g und 4°C und der Überstand weiterbehandelt.

Das Verfahren zur Herstellung des nicht-viralen Vektorsystems zum Transport von Nukleinsäuren beinhaltet die Herstellung eines Polymerkonjugates aus PEG und PEI und die Umsetzung mit Peptidsequenzen mit PTD/CPP-Funktionalität beispielsweise dem TAT-Peptid oder TAT-ähnlichem Peptid.

Die TAT-PEG-PEI Polyplexe aus PEI, beispielsweise einem 25 kDa verzweigten PEI, einem Linker aus PEG und einer TAT-ähnlichen Oligopeptidsequenz beipielsweise GRKKKRRQRC werden nach folgendem Reaktionsschema hergestellt:
i) Umsetzung von bifunktionalem verzweigtem Polyethylenglykol (PEG) mit einer α-Vinyl-Sulfon- und einer ω-N-Hydroxy-succinimid-Estergruppe (NHS-PEG-VS) mit Polyethylenimin (PEI) zu aktiviertem PEI (VS-PEG-PEI)
ii) Reaktion des aktivierten PEI (VS-PEG-PEI) mit einem Peptid mit PTD/CPP-Funktionalität
iii) Abtrennung der Polymerkomplexe von nicht-gebundenem PEG und niedermolekularen Resten

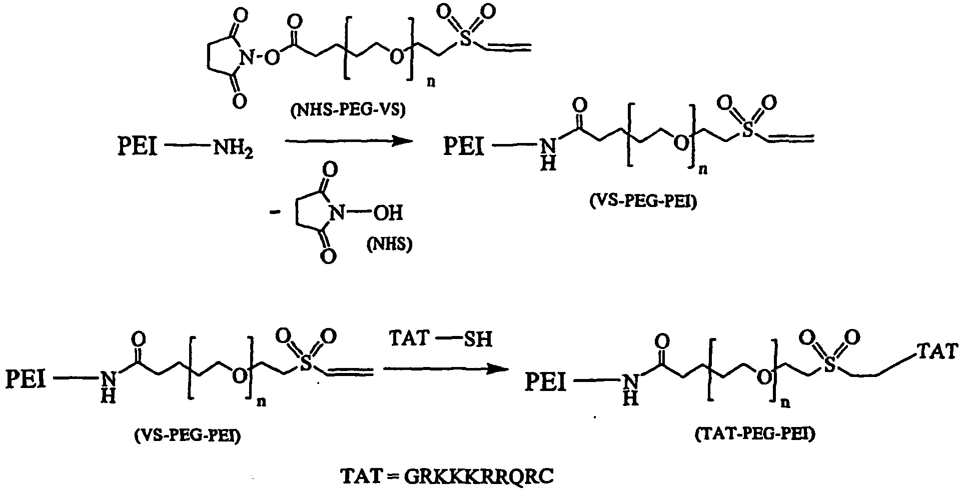

Ausgangsstoff des Verfahrens ist bifunktionales verzweigtes Polyethylenglykol (PEG) mit einer α-Vinyl-Sulfon- und einer ω-N-Hydroxy-succinimid-Estergruppe (NHS-PEG-VS), wie es beispielsweise kommerziell von der Firma Nektar Therapeutics (Huntsville, USA) erhältlich ist.

NHS-PEG-VS wird mit Polyethylenimine (PEI), das kommerziell z.B. bei BASF erhältlich ist, bei pH 5.5 umgesetzt.

Das so aktivierte PEI (VS-PEG-PEI) wird in einer weiteren Reaktion mit dem TAT-ähnlichen Peptid durch die Umsetzung via SH-Gruppe gekoppelt.

### Aktivierung von PEI

19.2 mg (565 µmol) bifunktionales PEG (3.4 kDa) mit einer α-Vinylsulfon- und einer ω-N-hydroxysuccinimid-Gruppe werden in einem Kolben vorgelegt. 4.293 ml einer PEI Lösung (entspricht 12.15 mg/0.486 µmol PEI; 282.4 µmol total Amin in 0.1 M Boratpuffer pH 5.5) werden hinzugefügt und gerührt. Die Aktivierungsreaktion wird für 4 Stunden bei Raumtemperatur weitergeführt. Der pH-Wert wird mit 1 N Salzsäure auf 7 eingestellt und die Reaktion zusätzlich 2 Stunden bei Raumtemperatur weitergeführt.

### Kopplung des Oligonukleotides an das aktivierte PEG-PEI

Für die Kopplung werden 2.98 mg (2.26 µmol) des Decapeptids GRKKKRRQRC in 866 µl Wasser gelöst. Die Peptidlösung wird zu dem aktivierten PEG-PEI hinzugefügt und die Reaktion 2 Stunden bei Raumtemperatur weitergeführt.

### Reinigung des TAT-PEG-PEI

Eventuell nicht gebundenes PEG oder Peptid und niedermolekulare Reste werden entfernt, z.B. durch eine Ultrafiltrationszelle (Amicon, Bedford, USA) mit einer 10 kDa Molekulargewichtsausschlussmembran (Millipore, Bedford, USA) und eluiert mit 0.1 M Boratpuffer bei pH 7.5.

### Komplexbildung

Zur Komplexherstellung wird Konjugat bzw. DNA getrennt mit 5%iger Glucoselösung pH 7.4 auf die gewünschte Konzentration verdünnt.

Dann wird die Polymerlösung der Nukleinsäure beispielsweise DNA hinzugefügt und durch schnelles auf und abpipettieren gut gemischt. Es folgt eine Inkubationszeit von 10-20 Minuten bei Raumtemperatur.

### Messung der Kondensationsfähigkeit im Ethidiumbromid-Ausschlussassay

Das Quenching der Ethidiumbromid (EtBr)-Fluoreszenz ist ein Maß für die Kondensationsfähigkeit der DNA.

Dazu wird in 96-well Platten je 8 µg Herings- oder Lachs-Testes-DNA in 60 mM Trispuffer pH 7.4 mit steigenden Mengen an PEI oder TAT-PEG-PEI in ingesamt 280 µL Puffer komplexiert. Nach 10 min Inkubationszeit wird 20 µl EtBr-Lösung (0.1 mg/ml) hinzugefügt. Die Fluoreszenz wird bei λₑₓ = 518 nm und λₑₘ = 605 nm gemessen z.B. mit einem Perkin Elmer LS 50 B Fluoreszenz-Plattenleser.

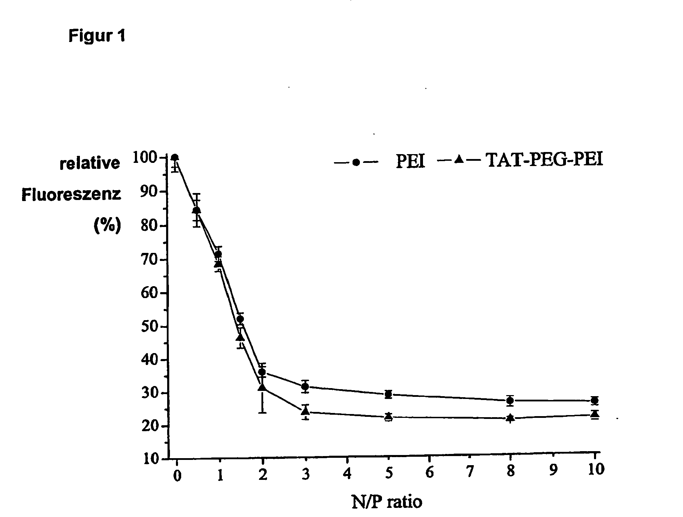

Freies Ethidiumbromid zeigt an sich schwache Fluoreszenz. Diese Fluoreszenz steigt, sobald es in die DNA interkaliert. Figur 1 zeigt den Ethidiumbromid-Ausschluss-Aassay, darin ist das Quenchen der DNA/Ethidiumbromidfluoreszenz dargestellt als "relative fluorescence", dabei entsprechen 100% der Kontrolle aus DNA mit Ethidiumbromid ohne Polymer. Dargestellt ist das Quenchen für PEI (schwarzer Kreis) und TAT-PEG-PEI (schwarzes Dreieck) in Gegenwart von Lachs-Testes-DNA. Die Werte sind dargestellt als Mittelwert ± SD (n = 4). Es wird deutlich, dass TAT-PEG-PEI ein stärkeres Quenchen der Fluoreszenz und damit stärkere Kondensationsfähigkeit aufweist als reines PEI 25kDa.

### Physikalisch-chemische Eigenschaften

Wichtige Parameter für einen verbesserten Gentransfer in Zellen sind das Zeta-Potential und die Partikelgröße der erfindungsgemäßen Polyplexe. Die Endocytose von Partikeln steigt bei steigendem Zeta-Potential, gleichzeitig verstärken sich aber auch die toxischen Nebeneffekte durch unspezifische Interaktionen zwischen den Partikeln und der Zellmembran.

Wünschenswert sind daher Partikel zum Gentransfer in Zellen mit einem stark positiven Zeta-Potential bei gleichzeitig geringer Toxizität.

Der Gentransfer in Lungenzellen ist zusätzlich von weiteren Schwierigkeiten charakterisiert, z.B. durch eine Mukusschicht, welche den Zellkontakt von Polyplexen hemmt und zur Entfernung von Partikeln aus der Lunge führt (Mukoziliäre Clearance). Ferner ist die Phagozytose durch alveolare Makrophagen eine Verlustquelle für Polyplexe. Die endozytotische Aufnahme der Partikel ist insgesamt abhängig von der Partikelgröße, diese steigt, mit Abnahme des Partikeldurchmessers. Wünschenswert sind daher Partikel zum Gentransfer in Lungenzellen, die klein und auch bei verschiedenen N/P Verhältnissen in verschiedenen Medien stabil sind. Dem Fachmann ist bekannt, dass der N/P Wert das Verhältnis von DNA zu PEI durch die Angabe des molaren Verhältnisses der Stickstoffatome im PEI zu den Phosphatatomen in der DNA angibt.

Die erfindungsgemäßen TAT-PEG-PEI-Polyplexe erfüllen diese Anforderungen als nicht-virales Vektorsystem zum Transport von Nukleinsäuren.

Die Messung der Oberflächenladung und des korrespondierenden Zeta-Potentials erfolgt mittels Laser Doppler Anemometrie. Nackte DNA hat ein stark negatives Zeta-Potential von -33 mV, die erfindungsgemäßen Komplexe zeigen dagegen ein positives Zeta-Potential. Für TAT-PEG-PEI-Polyplexe wird ein Zeta-Potential von 15 ± 3 mV bis 20 ± 3 mV (abhängig vom NP-Verhältnis) beobachtet, PEI 25kDa-Komplexe zeigen ein Zeta-Potential von 32 mV.

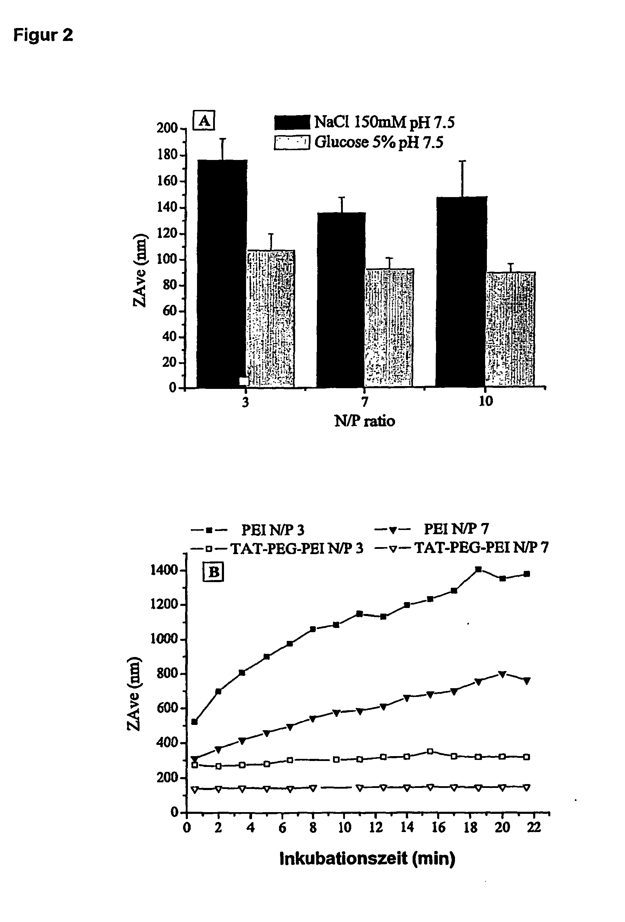

Figur 2 zeigt die Ergebnisse der Messung der Partikelgröße von TAT-PEG-PEI-Polyplexen und Plasmid-DNA im Vergleich.

Teil A zeigt hydrodynamische Durchmesser der TAT-PEG-PEI/pGL3-Polyplexe in verschiedenen Medien. Der dunkle Balken bezieht sich auf die Ergebnisse in NaCl 150 mM pH 7.5, der hellere Balken auf Ergebnisse in Glucose 5% pH 7.5. Teil B zeigt den Vergleich von TAT-PEG-PEI-Polyplexen zu PEI-Polyplexen in ihrer Tendenz in stark ionischem Medium (NaCl 150 mM bei pH 7.5) Aggregate zu bilden. Verglichen werden PEI 25kDa-Polyplexe hergestellt bei N/P 3 (schwarze Quadrate), PEI 25kDa Polyplexe bei N/P-Verhältnis 7 (schwarze Dreiecke), TAT-PEG-PEI-Polyplexe bei N/P-Verhältnis 3 (weiße Quadrate) und TAT-PEG-PEI-Polyplexe bei N/P-Verhältnis 7 (weiße Dreiecke).

Die erfindungsgemäßen Polyplexe weisen in 150 mM NaCl-Lösung Größen von 135 bis 176 nm auf, in Glucose 5% Größen von 89 bis 107 nm bei pH 7.5. Eine geringere Partikelgröße wird für TAT-PEG-PEI/DNA in Glucose-Lösung beobachtet wenn das N/P-Verhältnis von 3 auf 10 ansteigt. Polyplexe, die mit einem N/P Verhältnis von 3 präpariert werden, weisen eine höhere Partikelgröße auf.

Die in Medium mit hoher Ionenstärke (150 mM NaCl) hergestellten TAT-PEG-PEI-Polyplexe sind über 20 Minuten stabil, während PEI Polyplexe als Kontrolle einen drastischen Anstieg der Partikelgröße in Folge von Aggregation zeigen.

### Stabilität der Polyplexe

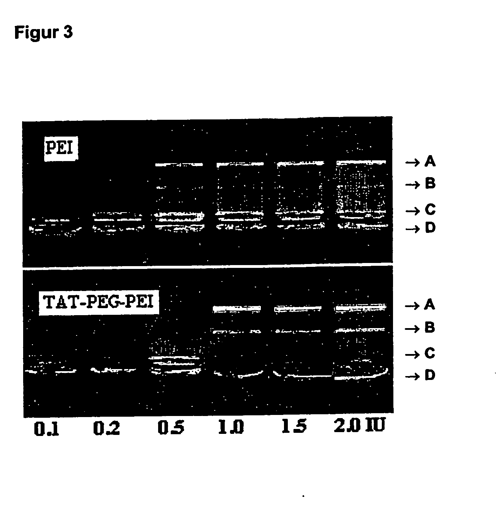

TAT-PEG-PEI Polyplexe mit und ohne DNA werden hinsichtlich der Komplexstabilität bezüglich Polyanionen und enzymatische Degradation in der Lungenumgebung untersucht. Die Stabilität der Polyplexe wird experimentell in Gegenwart von Heparin, Alveofact®, BALF und DNase I (Figur 3-5) gezeigt.

Dazu werden die Polyplexe (N/P Verhältnis 8) mit steigenden Mengen an Heparin versetzt (Figur 3). PEI schützt die Plasmid-DNA gegen Austausch durch Heparin bis zu 0.2 IU Heparin. Bei einer Menge von 0.5 IU Heparin erfolgt die Freisetzung der DNA von den PEI-Polyplexen. TAT-PEG-PEI-Polyplexe sind auch in Gegenwart von 0.5 IU Heparin stabil und lassen keine DNA frei.

Figur 3 zeigt die Ergebnisse dieser Versuche zur Stabilität der Polyplexe in Heparin. PEI und TAT-PEG-PEI-Polyplexe (N/P Verhältnis 8) werden mit steigenden Mengen an Heparin versetzt (0.1, 0.2, 0.5, 1, 1.5, 2, IU Heparin per 1 µg pGL3). Die verschiedenen morphologischen Formen der DNA sind bezeichnet mit A: supercoiled, B: linear und C: open circular. D gibt die Startlinie an.

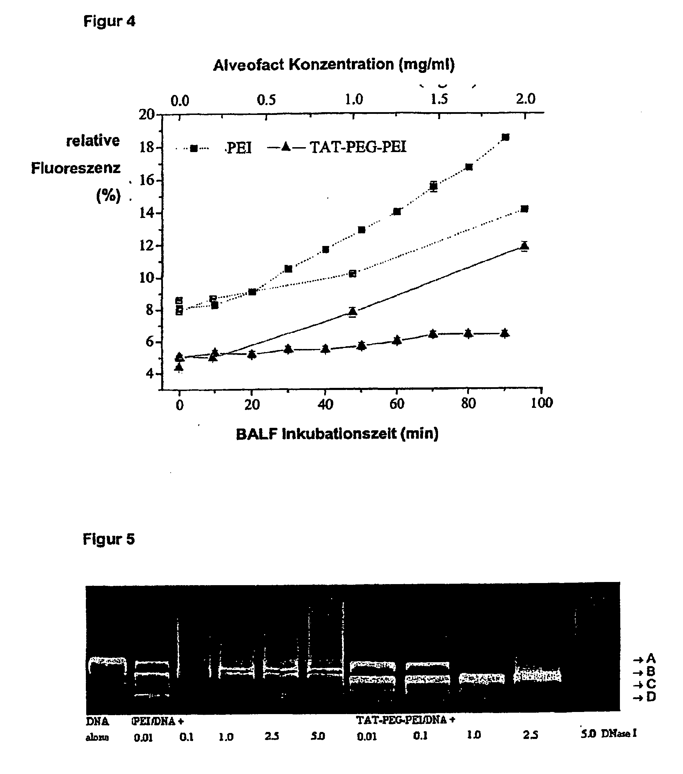

Die Stabilitätsstudien mit Alveofact^{®} und BALF werden mit dem reversen EtBr Ausschlussassay durchgeführt und sind in Figur 4 gezeigt. Ohne Alveofact^{®} und BALF, zeigt sich die Fähigkeit zur Kondensation der DNA bis zu einer Fluoreszenz von 8 % (PEI) und 5 % (TAT-PEG-PEI). Zugabe von Alveofact^{®} steigert die Fluoreszenz für beide Polyplexe konzentrationsabhängig. PEI/DNA zeigt bei einer Alveofact(8)-Konzentration von 2 µg/µl eine Stabilität von 14.1 % und TAT-PEG-PEI-Polyplexe zeigen eine Stabilität von 11.8 %. Die Fluoreszenz, die mit der DNA-Freigabe ansteigt, der PEI-Polyplexe in BALF steigt linear von 8 % (0 min) bis zu 18.5 % relative Fluoreszenz (90 min), die Fluoreszenz von den TAT-PEG-PEI-Polyplexen (1,4 %) steigt nicht an innerhalb der Beobachtungszeit von 90 min. Dies zeigt, dass die DNA in den Polyplexen TAT-PEG-PEI gegen extrazelluläre pulmonare Enzyme und Proteolipide geschützt ist.

Figur 4 zeigt die Ergebnisse nach Behandlung von PEI und TAT-PEG-PEI-Polyplexen mit N/P-Verhältnis von 8 mit steigenden Mengen an Alveofact^{®} (graue Linie) und BALF (schwarze Linie).

Figur 5 zeigt die Ergebnisse der DNA-Abbaustudie mit der Nuclease DNase 1. TAT-PEG-PEI-Polyplexe werden mit steigenden Konzentrationen an DNase I über 15 Minuten versetzt DNA-Spaltung erfolgt bei einer Konzentration von 2.5 IU DNase I per 1 µg DNA. Erst bei über 5 IU wird die gesamte Plasmid DNA freigesetzt.

Neben der Stabilität gegenüber intrazellulären Enzymen (z.B. in Endosomen, Lysosomen) ist das erfindungsgemäße nicht-virale Vektorsystem für den Transport von Nukleinsäure auch sehr stabil gegenüber extrazellulärer Umgebung und damit zum effizienten Einsatz in der Lunge geeignet. Verglichen mit PEI ist die Stabilität der TAT-PEG-PEI-Polyplexe in Gegenwart von hohen Konzentrationen an Heparin, Alveofact^{®}, BALF und DNase I signifikant höher.

### Transfektionseffizienz

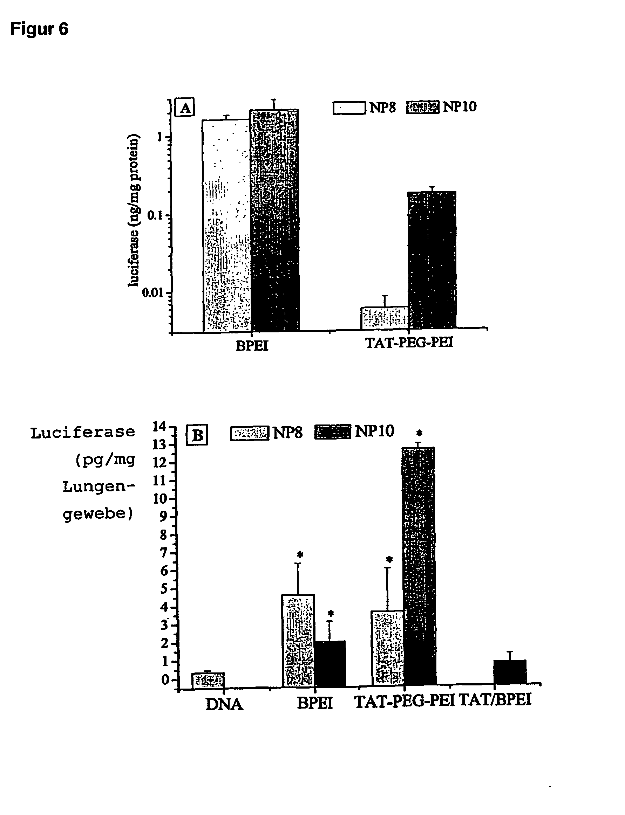

Um die Transfektionseffizienz zu untersuchen wird Plasmid-DNA komplexiert mit PEI oder TAT-PEG-PEI (N/P-Verhältnis 8 und 10). Figur 6A zeigt die in vitro Ergebnisse und Figur 6B die in vivo Ergebnisse an der Mäuselunge.

Während die Transfektionseffizienz der TAT-PEG-PEI-Polyplexe in Lungenepithelzellen A549 niedriger ist als die von PEI 25kDa-Polyplexen, erfolgt eine starke Genexpression in den Versuchen mit Mäuselunge. Bei N/P-Verhältnis 10, zeigt sich eine starke Genexpression des TAT-PEG-PEI (12.6 pg Luciferase/mg Lungengewebe) eine geringere für PEI (2 pg Luciferase/mg Lungengewebe). Insgesamt zeigen TAT-PEG-PEI-Polyplexe 600% Anstieg in der Transfektionseffizienz in vivo verglichen mit PEI.

### Toxizität

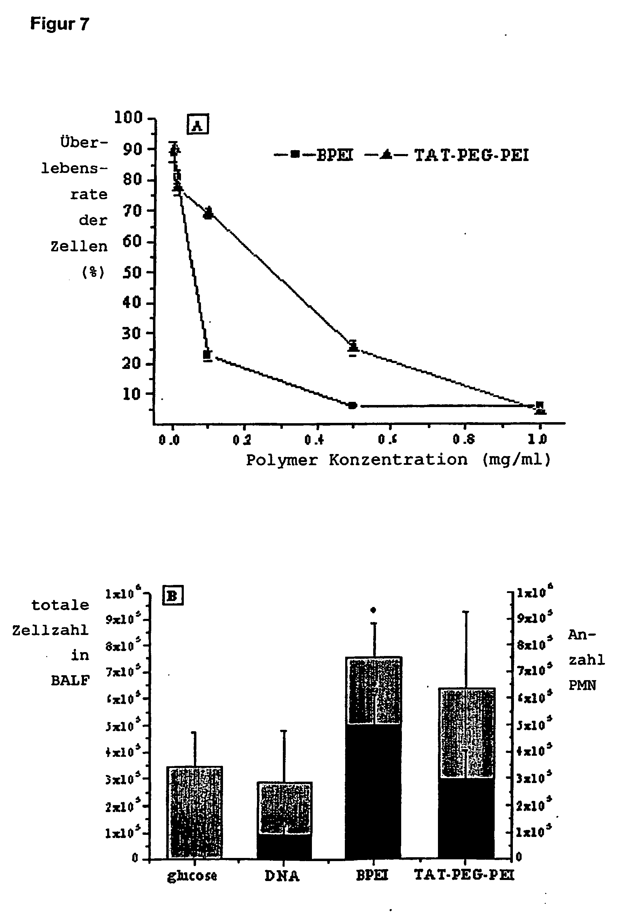

Die metabolische und mitochondriale Aktivität von Zellen wird mit dem kolorimetrischen MTT-Assay bestimmt. Figur 7A zeigt die Toxizität der Polymere konzentrationsabhängig. PEI reduziert die Lebensfähigkeit der Zellen bei Konzentrationen von 0.1 mg/ml (- 23 % Lebensfähigkeit), TAT-PEG-PEI-Polyplexe zeigen wenig Einfluss auf die Zellen (~ 70 % Lebensfähigkeit). IC₅₀ Werte liegen für BPEI: 0.071 mg/ml, TAT-PEG-PEI: 0.2 mg/ml.

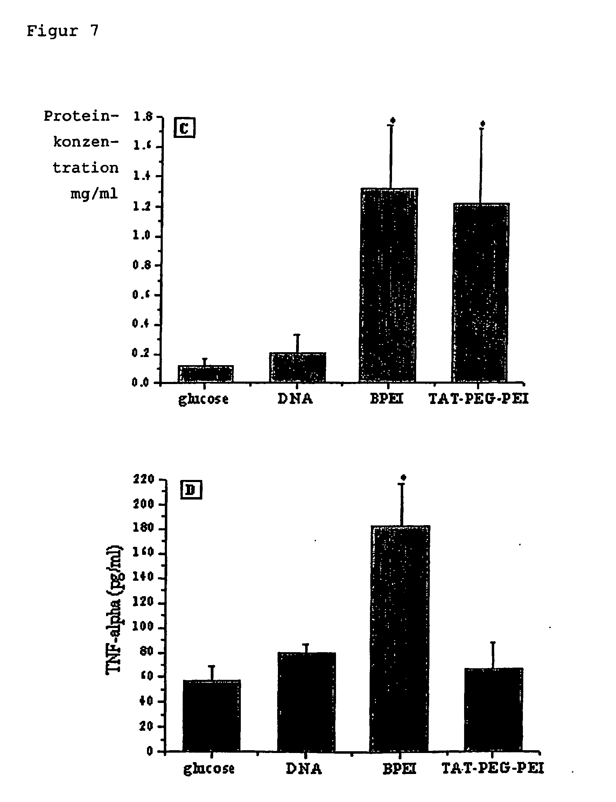

Figur 7B zeigt die Zellzahl der PEI-Polyplexe nach Behandlung in der Lunge, Figur 7C zeigt die Gesamtproteinkonzentration in BALF und Figur 7D zeigt die Experimente zur Steigerung des TNF-α Levels.

### Verteilung der Polyplexe in der Mäuselunge

Zur Bestimmung der Verteilung der Polyplexe wird doppelt markiertes TAT-PEG-PEI/Plasmid-Polyplex der Mäuselunge zugeführt und die Verteilung in Formaldehyd fixierten Kryoschnitten vier Stunden nach Applikation gemessen. Plasmid-DNA und Polymer sind größtenteils co-lokalisiert. Die doppelt markierten Polyplexe befinden sich in den bronchialen Epithelzellen und in der alveolaren Region. Diese Ergebnisse bestätigen die gute Eignung der TAT-PEG-PEI-Polyplexe als Vektorsystem für verschiedene Erkrankungen der Lunge.

### Abbildungsverzeichnis

**Figur 1** zeigt das Quenchen der Ethidiumbromidfluoreszenz (relative fluorescence in %) in einem Assay. Dargestellt ist das Quenchen für PEI (schwarzer Kreis) und TAT-PEG-PEI (schwarzes Dreieck) in Gegenwart von Lachs-Testes-DNA. Die Werte sind dargestellt als Mittelwert ± SD (n = 4).
**Figur 2** zeigt Ergebnisse bezüglich der Partikelgröße
   Teil A zeigt hydrodynamische Diameter der TAT-PEG-PEI/pGL3-Polyplexe in verschiedenen Medien. Der dunkle Balken bezieht sich auf die Ergebnisse in NaCl 150 mM pH 7.5, der hellere Balken auf Ergebnisse in Glucose 5% pH 7.5.
   Teil B zeigt den Vergleich von TAT-PEG-PEI-Polyplexen zu PEI-Polyplexen in ihrer Tendenz in stark ionischem Medium (NaCl 150 mM bei pH 7.5) Aggregate zu bilden. Verglichen werden PEI-Polyplexe mit N/P ratio 3 (schwarze Quadrate), PEI-Polyplexe mit N/P-Verhältnis 7 (schwarze Dreiecke), TAT-PEG-PEI-Polyplexe mit N/P-Verhältnis 3 (weiße Quadrate) und TAT-PEG-PEI-Polyplexe mit N/P-Verhältnis 7 (weiße Dreiecke).
**Figur 3** zeigt die Stabilität der Polyplexe in Heparin.
   PEI und TAT-PEG-PEI-Polyplexe (N/P-Verhältnis 8) werden mit steigenden Mengen an Heparin versetzt (0.1, 0.2, 0.5, 1, 1.5, 2, IU Heparin per 1 µg pGL3).
   Die verschiedenen DNA-Morphologien sind bezeichnet mit A: supercoiled, B: linear und C: open circular. D gibt die Startlinie an.
**Figur 4** zeigt die Ergebnisse nach Behandlung von PEI und TAT-PEG-PEI-Polyplexen (N/P Verhältnis 8) mit steigenden Mengen an Alveofact^{®} (graue Linie) und BALF (schwarze Linie). Die Werte sind mit Standardabweichung ± SD angegeben und als Prozent der maximalen Fluoreszenz angegeben (pGL3 alleine).
**Figur 5** zeigt PEI und TAT-PEG-PEI-Polyplexe (N/P-Verhältnis 8) und Plasmid-DNA mit steigenden Mengen an DNase I (Bild oben: PEI 25kDa, Bild unten :TAT-PEG-PEI-Polyplexe) (0.01, 0.1, 1, 2.5 and 5 IU DNase I per 1 µg pGL3) Spur 1 zeigt die Kontrolle freie, ungespaltene pGL3. Die verschiedenen DNA-Morphologien sind bezeichnet mit A: supercoiled, B: linear und C: open circular. D gibt die Startlinie an.
**Figur 6** zeigt die Transfektionseffizienz der Polyplexe bei N/P-Verhältnis 8 und 10: A) Luciferase-Expression in A549, B) Luciferase-Expression in C57BU6 Mäuselunge. Die in-vivo Resultate sind mit ± SD dargestellt.
**Figur 7** zeigt die Ergebnisse der Toxizitätsstudien: A) Zytotoxische Effekte der Polymere bei Konzentrationen von 0.001, 0.01, 0.1, 0.5, und 1.0 mg/ml an A549 Zellen, bestimmt durch MTT-Assay und dargestellt als relative Zellviabilität (Werte ± SD von 6 Messungen). Pulmonale Inflammationsindikatoren (Werte ± SD von 3 Messungen) in BALF 24 h nach Applikation der Polyplexe (N/P 10) in der Mäuselunge: B) Gesamte Zellen und PMN Nummer, C) Gesamt-Proteinkonzentration, D) TNF-alpha-Konzentration.

## Patentansprüche

1. Nicht-virales Vektorsystem für den Transport von Nukleinsäuren, wobei das Vektorsystem ein Polymerkonjugat aus Polyethylenimin (PEI), Polyethylenglykol (PEG) als hetero-bifunktionalem Linker und einem Peptid, wobei das Peptid über den hetero-bifunktionalen Linker kovalent an das Poylethylen (PEI) gekoppelt ist, **dadurch gekennzeichnet dass** das Peptid eine Proteintransduktionsdomäne (PTD) oder eine Zellpenetrationsdomäne (CPP) aufweist und dass das Peptid ein Oligopeptid ist, welches am C-Terminus über einen Cysteinrest modifiziert und über den hetero-bifunktionalen Linker kovalent an das Polyethylenimin (PEI) gekoppelt ist, wobei das Peptid ein TAT-Peptid ist und wobei das Peptid die Decapeptidsequenz GRKKKRRQRC hat.

2. Nicht-virales Vektorsystem entsprechend Anspruch 1, **dadurch gekennzeichnet, dass** das nicht-virale Vektorsystem ein Polymerkonjugat ist, das einen als Polyplex bezeichneten Komplex mit Nukleinsäure bildet.

3. Nicht-virales Vektorsystem entsprechend einem der Ansprüche 1 oder 2, **dadurch gekennzeichnet, dass** die Polyplexe in 150 mM NaCl-Lösung eine Größe von 135 bis 176 nm aufweisen und in 5%-Glucose eine Größe von 89 bis 107 nm aufweisen.

4. Nicht-virales Vektorsystem entsprechend einem der Ansprüche 1 bis 3, **dadurch gekennzeichnet, dass** es wenigstens eine eingeschlossen Nukleinsäure aufweist.

5. Nicht-virales Vektorsystem entsprechend Anspruch 4, **dadurch gekennzeichnet, dass** die Nukleinsäure DNA oder RNA ist.

6. Nicht-virales Vektorsystem entsprechend wenigstens einem der Ansprüche 1 bis 5 **dadurch gekennzeichnet, dass** es hergestellt ist durch ein Verfahren umfassend die Schritte
a. Umsetzung von Polyethylenimin mit bifunktionalem verzweigtem Polyethylenglykol, umfassend eine α-Vinyl-Sulfon- und eine ω-N-Hydroxy-succinimid-Estergruppe (NHS-PEG-VS), zu aktiviertem PEG-PEI (VS-PEG-PEI);
b. Kopplung des Oligopeptides an das aktivierte PEG-PEI
c. Reinigung des Peptid-PEG-PEI-Konjugates
d. Komplexbildung.

7. Nicht-virales Vektorsystem nach Anspruch 6, **dadurch gekennzeichnet dass** die Kopplung des Oligopeptides an das aktivierte PEG-PEI durch Reaktion des aktivierten PEI (VS-PEG-PEI) mit einem Peptid mit einer Proteintransduktionsdomäne (PTD) oder einer Zellpenetrationsdomäne (CPP) erfolgt, wobei das Peptid C-terminal über eine aktivierte Cystein-Gruppe an die Vinyl-Sulfongruppe des bifunktionalen Linkers PEG gekoppelt wird.

8. Nicht-virales Vektorsystem nach einem der Ansprüche 6 oder 7, **dadurch gekennzeichnet, dass** die Reinigung des Peptid-PEG-PEI-Konjugates durch Entfernung von nicht-gebundenem PEG und niedermolekularen Resten erfolgt, wobei die folgenden Schritte ausgeführt werden:
a. Ultrafiltration mit einer Ultrafiltrationszelle mit einer 10 kDa Molekulargewichtsausschlussmembran und
b. Elution mit 0.1 M Boratpuffer bei pH 7.5.

9. Verfahren zur Herstellung eines nicht-viralen Vektorsystems entsprechend wenigstens einem der Ansprüche 1 bis 5, umfassend die Schritte
a. Umsetzung von Polyethylenimin mit bifunktionalern verzweigtem Polyethylenglykol, umfassend eine -α-Vinyl-Sulfon- und eine ω-N-Hydroxy-succinimid-Estergruppe (NHS-PEG-VS), zu aktiviertem PEG-PEI (VS-PEG-PEI);
b. Kopplung des Oligopeptides an das aktivierte PEG-PEI
c. Reinigung des Peptid-PEG-PEI-Konjugates
d. Komplexbildung.

10. Verfahren nach Anspruch 9, **dadurch gekennzeichnet, dass** die Kopplung des Oligopeptides an das aktivierte PEG-PEI durch Reaktion des aktivierten PEI (VS-PEG-PEI) mit einem Peptid mit einer Proteintransduktionsdomäne (PTD) oder einer Zellpenetrationsdomäne (CPP) erfolgt, wobei das Peptid C-terminal über eine aktivierte Gystein-Gruppe an die Vinyl-Suffongruppe des bifunktionalen Linkers PEG gekoppelt wird.

11. Verfahren nach einem der Ansprüche 9 oder 10, **dadurch gekennzeichnet, dass** die Reinigung des Peptid-PEG-PEI-Konjugates durch Entfernung von nicht-gebundenem PEG und niedermolekularen Resten erfolgt, wobei die folgenden Schritte ausgeführt werden:
a. Ultrafiltration mit einer Ultrafiltrationszelle mit einer 10 kDa Molekulargewichtsausschlussmembran und
b. Elution mit 0.1 M Boratpuffer bei pH 7.5.

12. Verfahren nach einem der Ansprüche 9 bis 11, **dadurch gekennzeichnet dass** die Komplexbildung die Schritte umfasst
a. Verdünnung des Konjugats mit einer 5%iger Glucoselösung, die einen pH-Wert von 7.4 hat, auf die gewünschte Konzentration und Verdünnung der DNA mit einer 5%iger Glucoselösung, die einen pH-Wert von 7.4 hat, auf die gewünschte Konzentration,
b. Vermischung der verdünnten Konjugat-Lösung mit der Nukleinsäure
c. Inkubation für 10 bis 20 Minuten bei Raumtemperatur.

13. Verwendung eines Peptides umfassend die Sequenz GRKKKRRQRC zur Herstellung eines nicht-viralen Vektorsystems gemäß einem der Ansprüche 1 bis 5.

14. Verwendung entsprechend Anspruch 13, **dadurch gekennzeichnet dass** die Herstellung des nicht-viralen Vektorsystems mit Hilfe des Verfahrens nach einem der Ansprüche 10 bis 13 erfolgt.

## Claims

1. A non-viral vector system for the transport of nucleic acids, wherein the vector system is a polymer conjugate made from polyethyleneimine (PEI), polyethylene gylcol (PEG) as a bifunctional linker and a peptide, wherein the peptide is covalently bonded to the polyethylene (PEI) via the hetero-bifunctional linker, wherein the peptide comprises a protein transduction domain (PTD) or a cell penetration domain (CPP), and the peptide is an oligopeptide which is modified at the C-terminus via a cysteine residue, and is covalently bonded to the polyethyleneimine (PEI) via the heterobifunctional linker, wherein the peptide is a TAT peptide and wherein the peptide has the decapeptide sequence GRKKKRRQRC.

2. A non-viral vector system according to claim 1, wherein the non-viral vector system is a polymer conjugate which forms a complex with nucleic acid referred to as polyplex.

3. A non-viral vector system according to one of the claims 1 or 2, wherein the polyplexes comprise a size of 135 to 176nm in a 150 mM NaCl solution und comprise a size of 89 to 107nm in 5% glucose.

4. A non-viral vector system according to one of the claims 1 to 3, wherein it comprises at least one enclosed nucleic acid.

5. A non-viral vector system according to claim 4, wherein the nucleic acid is DNA or RNA.

6. A non-viral vector system according to at least one of the claims 1 to 5, wherein it is produced by a method comprising the steps
a. reaction of polyethyleneimine with bifunctionally branched polyethylene glycol, comprising an α-vinyl-sulfone and an ω-N-hydroxysuccinimide ester group (NHS-PEG-VS) into activated PEG-PEI (VS-PEG-PEI);
b. bonding of oligopeptides to the activated PEG-PEI
c. purification of the peptide PEG-PEI conjugate
d. complex formation.

7. A non-viral vector system according to claim 6, wherein the oligopeptide is bonded to the activated PEG-PEI by reacting the activated PEI (VS-PEG-PEI) with a peptide with a protein transduction domain (PTD) or a cell penetration domain (CPP), wherein the peptide is bonded at the C-terminus to the vinyl sulfonic group of the bifunctional linker PEG via an activated cysteine group.

8. A non-viral vector system according to one of the claims 6 or 7, wherein the purification of the peptide PEG-PEI conjugate takes place by removing non-bonded PEG and low-molecular residues, wherein the following steps are carried out:
a. ultrafiltration with an ultrafiltration cell with a 10 kDa molecular weight exclusion membrane, and
b. elution with a 0.1 M borate buffer at pH 7.5.

9. Method for the production of a non-viral vector system according to at least one of the claims 1 to 5 comprising the steps
a. reaction of polyethyleneimine with bifunctionally branched polyethylene glycol, comprising an α-vinyl-sulfone and an ω-N-hydroxysuccinimide ester group (NHS-PEG-VS) into activated PEG-PEI (VS-PEG-PEI);
b. bonding of oligopeptides to the activated PEG-PEI
c. purification of peptide PEG-PEI conjugate
d. complex formation.

10. Method according to claim 9, wherein the oligopeptide is coupled to the activated PEG-PEI by reacting the activated PEI (VS-PEG-PEI) with a peptide with a protein transduction domain (PTD) or a cell penetration domain (CPP), wherein the peptide is bonded at the C-terminus to the vinyl sulfonic group of the bifunctional linker PEG via an activated cysteine group.

11. Method according to one of the claims 9 or 10, wherein the purification of the peptide PEG-PEI conjugate takes place by removing non-bonded PEG and low-molecular residues, wherein the following steps are carried out:
a. ultrafiltration with an ultrafiltration cell with a 10 kDa molecular weight exclusion membrane, and
b. elution with a 0.1 M borate buffer at pH 7.5.

12. Method according to one of the claims 9 to 11, wherein the complex formation comprises the steps
a. dilution of the conjugate with a 5% glucose solution with a pH value of 7.4 to the desired concentration, and dilution of the DNA with a 5% glucose solution with a pH value of 7.4 to the desired concentration,
b. mixing of the diluted conjugate solution with the nucleic acid,
c. incubation for 10 to 20 minutes at room temperature.

13. Use of a peptide comprising the sequence GRKKKRRQRC for the production of a non-viral vector system according to one of the claims 1 to 5.

14. Use according to claim 13, wherein the production of the non-viral vector system takes place with the help of the method according to one of the claims 10 to 13.

## Revendications

1. Système de vecteur non-viral pour le transport des acides nucléiques, où le système de vecteur est un conjugué polymère de polyéthylènimine (PEI), un polyéthylène glycol (PEG) comme agent de liaison hétérobifonctionnel et un peptide, où le peptide est lié par l'intermédiaire de l'agent de liaison hétérobifonctionnel de manière covalente au polyéthylène (PEI), **caractérisé en ce que**, le peptide comporte un domaine de transduction de protéines (PTD) ou un domaine de pénétration cellulaire (CPP) et que le peptide est un oligopeptide, qui qui est modifié au C-terminal par un résidu cystéine et est lié par l'intermédiaire de l'agent de liaison hétérobifonctionnel de manière covalente au polyéthylène (PEI), où le peptide est un peptide TAT et où le peptide a la séquence décapeptide GRKKKRRQRC.

2. Système de vecteur non-viral selon la revendication 1, **caractérisé en ce que** le système de vecteur non-viral est un conjugué de polymère qui forme avec l'acide nucléique, un complexe indiqué comme polyplexe.

3. Système de vecteur non-viral selon une des revendications 1 ou 2, **caractérisé en ce que** les polyplexes, dans une solution à 150 mM de NaCI, présentent une dimension de 135 à 176 nm et dans 5% de glucose une dimension de 89 à 107 nm.

4. Système de vecteur non-viral selon l'une des revendications 1 à 3, **caractérisé en ce qu'**il comprend au moins un acide nucléique enfermé.

5. Système de vecteur non-viral selon la revendication 4, **caractérisé en ce que** l'acide nucléique est un DNA ou un RNA.

6. Système de vecteur non-viral selon au moins l'une des revendications 1 à 5, **caractérisé en ce qu'**il soit produit par un procédé comprenant les étapes
a. Réaction de polyéthylèneimine avec du polyéthylène glycol ramifié bifonctionnel comprenant un groupe ester α-vinylsulfone et un groupe ester ω-N-hydroxy-succinimide (NHS-PEG-VS), au PEG-PEI (VS-PEG-PEI) activé
b. Liaison de l'oligopeptide au PEG-PEI activé
c. Nettoyage du conjugué peptide-PEG-PEI
d. Formation du complexe.

7. Système de vecteur non-viral selon la revendication 6, **caractérisé en ce que** la liaison de l'oligopeptide au PEG-PEI activé se fait par réaction du PEI activé (VS-PEG-PEI) avec un peptide avec un domaine de transduction de protéines (PTD) ou un domaine de pénétration cellulaire (CPP), où le peptide est lié par un groupe cystéine activé avec son C-terminal au groupe vinylsulfone de l'agent de liaison bifonctionnel PEG.

8. Système de vecteur non-viral selon l'une des revendications 6 ou 7, **caractérisé en ce que** le nettoyage du conjugué peptide-PEG-PEI s'effectue par élimination du PEG non-lié et des résidus de faible masse moléculaire, où les étapes suivantes sont réalisées :
a. Ultrafiltration avec une cellule d'ultrafiltration avec une membrane ayant un seuil de rétention moléculaire de 10 kDa et
b. Élution avec un tampon borate 0.1 M à pH 7,5.

9. Procédé de production d'un système de vecteur non-viral selon au moins l'une des revendications 1 à 5, comprenant les étapes :
a. Réaction de la polyéthylèneimine avec du polyéthylène glycol ramifié bifonctionnel comprenant un groupe ester α-vinylsulfone et un groupe ester ω-N-hydroxy-succinimide (NHS-PEG-VS), au PEG-PEI (VS-PEG-PEI) activé
b. Liaison de l'oligopeptide au PEG-PEI activé
c. Nettoyage du conjugué peptide-PEG-PEI
d. Formation du complexe

10. Procédé selon la revendication 9, **caractérisé en ce que** la liaison de l'oligopeptide au PEG-PEI activé se fait par réaction du PEI activé (VS-PEG-PEI) avec un peptide avec un domaine de transduction de protéines (PTD) ou un domaine de pénétration cellulaire (CPP), où le peptide est lié par un groupe cystéine activé avec son C-terminal au groupe vinylsulfone de l'agent de liaison bifonctionnel PEG.

11. Procédé selon une des revendications 9 ou 10, **caractérisé en ce que** le nettoyage du conjugué peptide-PEG-PEI s'effectue par élimination du PEG non-lié et des résidus de faible masse moléculaire, où les étapes suivantes sont réalisées :
a. Ultrafiltration avec une cellule d'ultrafiltration avec une membrane ayant un seuil de rétention moléculaire de 10 kDa et
b. Élution avec un tampon borate 0.1 M à pH 7,5.

12. Procédé selon l'une des revendications 9 à 11, **caractérisé en ce que** la formation du complexe comprend les étapes
a. Dilution du conjugué avec une solution de glucose à 5%, ayant un pH de 7,4, à la concentration souhaitée et une dilution du DNA avec une solution de glucose à 5%, ayant un pH de 7,4, à la concentration souhaitée,
b. Mélange de la solution de conjugué diluée avec l'acide nucléique
c. Incubation pendant 10 à 20 minutes à température ambiante.

13. Utilisation d'un peptide comprenant la séquence GRKKKRRQRC pour la production d'un système de vecteur non viral selon l'une des revendications 1 à 5.

14. Utilisation selon la revendication 13, **caractérisé en ce que** la production du système de vecteur non viral s'effectue avec l'aide du procédé selon une des revendications 10 à 13.
